# EUROPEAN PATENT SPECIFICATION

(11) **EP 4 527 292 B1**
(45) Date of publication and mention of the grant of the patent: **29.04.2026**
(21) Application number: 24202556.7
(22) Date of filing: 25.09.2024
(51) Int. Cl.: A61B 5/1473

(54) **BODY ATTACHABLE UNIT FOR CONTINUOUS GLUCOSE MONITORING SYSTEM**
AM KÖRPER ANBRINGBARE EINHEIT FÜR EIN KONTINUIERLICHES GLUCOSEÜBERWACHUNGSSYSTEM
UNITÉ POUVANT ÊTRE FIXÉE AU CORPS POUR SYSTÈME DE SURVEILLANCE CONTINUE DU GLUCOSE

(30) Priority: 25.09.2023 KR 20230128382
(43) Date of publication of application: 26.03.2025
(73) Proprietor: i-Sens, Inc., Seoul 06646 (KR)
(72) Inventor: CHO, Hee Gyung, 06646 Seoul (KR)
(74) Representative: BCKIP Part mbB

(56) References cited:
- EP-A1- 4 137 049
- WO-A1-2017/116915
- US-A1- 2021 290 116

## Description

### BACKGROUND

The present disclosure relates to a body-attachable unit. Specifically, the present disclosure relates to a body-attachable unit for improving convenience for manufacturing the body-attachable unit and reducing a product defect rate.

With recent development of a medical technology, various medical devices attached to a body of a user and used are developed and sold. A medical device attached to skin may be attached to a body of a patient with a chronic disease to monitor biometric information or be helpfully used in treatment.

For example, a chronic disease such as diabetes requires continuous care, and a medical device that is attached to a body to measure blood sugar may be used for monitoring blood sugar of a patient with diabetes. Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear. In order to diagnose diabetes and manage diabetes to prevent developing the complications, systematic measurement and treatment of the blood sugar may be done simultaneously.

For the patient with diabetes and people having sugar measured to be more than normal in blood, multiple medical device manufacturers provides various types of blood sugar meters for measuring the blood sugar.

A scheme of a blood sugar meter includes a scheme of gathering blood from a finger tip of the user to perform blood sugar measurement on a one-time basis and a scheme of being attached to a stomach and an arm of the user to continuously perform the blood sugar measurement.

In a case of the patient with diabetes, a hyperglycemia state and a hypoglycemia state generally alternate with each other. An emergency may occur in the hypoglycemia state, and the patient may lose consciousness or may die when the hypoglycemia state lasts long without supply of sugar. Thus, immediate detection of the hypoglycemia state is greatly important for the patient with diabetes. However, accurate identification thereof is limited with a blood gathering-type blood sugar meter that intermittently measures the blood sugar.

Recently, in order to overcome such limitation, a continuous glucose monitoring system (CGMS) that is inserted to measure a value of the blood sugar at intervals of several minutes is developed and used. In order to minimize a pain or an aversion of the user, which is caused by blood gathering, the continuous glucose monitoring system may insert a needle-shaped percutaneous sensor into a region such as a stomach and an arm at which the pain is relatively less and then continuously measure the blood sugar.

The continuous glucose monitoring system includes and is formed of a sensor unit including the percutaneous sensor inserted into skin of the user to measure the blood sugar in the body, a body-attachable unit including a transmitter for transmitting the value of the blood sugar which is measured by the percutaneous sensor, a terminal outputting the value of the blood sugar transmitted from the body-attachable unit, and the like.

A medical system using the percutaneous sensor is manufactured in various forms by each manufacturer, and a scheme of use thereof also varies. Recently, a variety of research and development is performed by each manufacturer for improving use convenience, measurement accuracy, and the like. However, continuous research and development is currently required for optimized quality.

Glucose monitoring systems according to the state of the art are for instance disclosed in US2021/0290116A1, EP4137049A1 and WO2017/116915A1.

### SUMMARY OF THE INVENTION

According to the invention, there is provided a body-attachable unit according to claim 1, preferred embodiments being specified in the dependent claims 2-6.

### SUMMARY OF THE DISCLOSURE

An aspect provides a body-attachable unit for improving convenience for manufacturing the body-attachable unit and reducing a product defect rate in a process of manufacturing the body-attachable unit which is inserted into a body to measure biometric information.

According to an aspect, there is provided a body-attachable unit configured to be attached to and inserted into a body for measuring biometric information, the body-attachable unit including a housing formed so that a bottom surface is configured to be attached to skin, a printed circuit board (PCB) substrate, which is disposed in the housing, with a plurality of electrical contacts formed to protrude from a surface of the PCB substrate, and a sensor member including a sensor body part disposed in the housing and a sensor probe part formed and extended from a side of the sensor body part to protrude outward from the bottom surface of the housing and configured to be inserted into the skin if the housing is attached to the skin, and at least one of the plurality of electrical contacts is formed to make contact with the sensor body part to support the sensor body part, remaining of the plurality of electrical contacts are formed to be spaced apart from the sensor body part, and the sensor body part is to make contact with all of the plurality of electrical contacts by manipulation by a user.

The body-attachable unit may further include a pressing operation module coupled to the housing to be operated by manipulation by the user and configured to press the sensor body part so that the sensor body part makes contact with all of the plurality of electrical contacts.

The sensor body part may transformed by pressing of the pressing operation module to make contact with all of the plurality of electrical contacts.

An electrical contact that makes contact with the sensor body part to support the sensor body part among the electrical contacts may be elastically transformed while maintaining a state of making contact with the sensor body part as the sensor body part is transformed by the pressing operation module.

The electrical contacts may be formed to elastically protrude from the PCB substrate in an elastically transformable shape.

An electrical contact that makes contact with the sensor body part to support the sensor body part among the electrical contacts may be formed to have a protrusion height from the PCB substrate higher than those of the remaining of the plurality of electrical contacts.

According to another aspect, there is also provided a body-attachable unit configured to be attached to and inserted into a body for measuring biometric information, the body-attachable unit including a housing formed so that a bottom surface is configured to be attached to skin, a printed circuit board (PCB) substrate, which is disposed in the housing, with a plurality of electrical contacts formed to protrude from a surface of the PCB substrate, and a sensor member including a sensor body part disposed in the housing and a sensor probe part formed and extended from a side of the sensor body part to protrude outward from the bottom surface of the housing and configured to be inserted into the skin if the housing is attached to the skin, and a sensor support member is formed to protrude from the PCB substrate to make contact with the sensor body part and configured to support the sensor body part so that the sensor body part is spaced apart from the electrical contacts, and the sensor body part is to make contact with all of the plurality of electrical contacts by manipulation by a user.

The sensor support member may be formed to elastically protrude from the PCB substrate in an elastically transformable shape.

The sensor support member may be formed to have a protrusion height from the PCB substrate higher than those of the plurality of electrical contacts.

The body-attachable unit may further include a pressing operation module coupled to the housing to be operated by manipulation by the user and configured to press the sensor body part so that the sensor body part makes contact with all of the plurality of electrical contacts, and the sensor body part may be transformed by pressing of the pressing operation module to make contact with all of the plurality of electrical contacts.

The sensor support member may be elastically transformed while maintaining a state of making contact with the sensor body part as the sensor body part is transformed by the pressing operation module.

Additional aspects of example embodiments will be set forth in part in the description which follows and, in part, will be apparent from the description, or may be learned by practice of the disclosure.

According to example embodiments, since a state in which a sensor member and an electrical contact are disposed in a process of manufacturing a body-attachable unit may be stably maintained, convenience for manufacturing the body-attachable unit may be improved, a product defect rate may be reduced.

### BRIEF DESCRIPTION OF THE DRAWINGS

These and/or other aspects, features, and advantages will become apparent and more readily appreciated from the following description of example embodiments, taken in conjunction with the accompanying drawings of which:
FIG. 1 is a perspective diagram illustrating an outline of an outward shape of an applicator assembly for a continuous glucose monitoring system according to the present disclosure;
FIG. 2 is a perspective diagram illustrating an outline of an outward shape of a body-attachable unit according to the present disclosure;
FIG. 3 is an exploded perspective diagram illustrating an outline of a configuration of an applicator according to the present disclosure;
FIG. 4 is a cross-sectional diagram taken along line B-B of FIG. 1;
FIG. 5 is a cross-sectional diagram taken along line A-A of FIG. 1;
FIGS. 6 through 10 are diagrams illustrating states of using a continuous glucose monitoring system according to the present disclosure by operation according to an operation order;
FIGS. 11 through 13 are diagrams illustrating an example in which a needle withdrawing elastic spring S2 is formed in a shape of a tension spring;
FIG. 14 is a perspective diagram illustrating an outline of an outward shape of a body-attachable unit attached to a body according to an example embodiment of the present disclosure;
FIG. 15 is an exploded perspective diagram illustrating an outline of a configuration of a body-attachable unit according to an example embodiment of the present disclosure;
FIG. 16 is a cross-sectional diagram taken along line C-C of FIG. 14;
FIG. 17 is a cross-sectional diagram taken along line D-D of FIG. 14;
FIG. 18 is a diagram illustrating an outline of an operation state of a pressing operation module according to an example embodiment of the present disclosure;
FIG. 19 is a diagram illustrating an outline of a detailed configuration of a pressing operation module according to an example embodiment of the present disclosure;
FIG. 20 is a diagram illustrating an outline of a detailed configuration of a sensor member according to an example embodiment of the present disclosure;
FIG. 21 is a diagram conceptually illustrating a state in which a sensor member is pressed to operate according to an example embodiment of the present disclosure;
FIG. 22 is a diagram conceptually illustrating a disposition relationship between a sensor member and an electrical contact according to an example embodiment of the present disclosure;
FIG. 23 is a diagram conceptually illustrating a disposition relationship between a sensor member and an electrical contact according to another example embodiment of the present disclosure;
FIG. 24 is a diagram conceptually illustrating a state in which a sensor member and an electrical contact illustrated in FIG. 23 are in contact; and
FIG. 25 is a diagram conceptually illustrating another form of a disposition structure of a sensor member and an electrical contact in a disposition relationship between the sensor member and the electrical contact according to another example embodiment of the present disclosure.

### DETAILED DESCRIPTION

Hereinafter, a desired example embodiment of the present disclosure will be described in detail with reference to the accompanying drawings. To begin with, it should be noted that, for adding a reference numeral to an element in each drawing, the same elements has the same reference numeral if possible although illustrated in different drawings. Also, for description of the present disclosure, a detailed description of an associated element or function known to the public will be omitted when determined as obscuring the gist of the present disclosure.

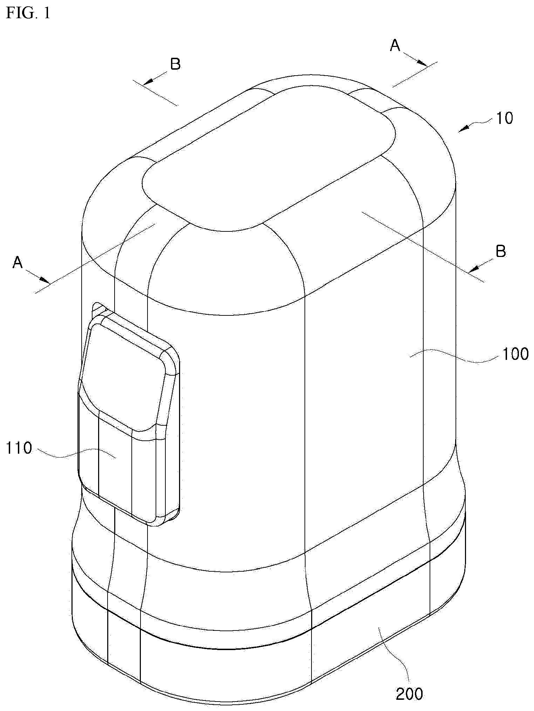

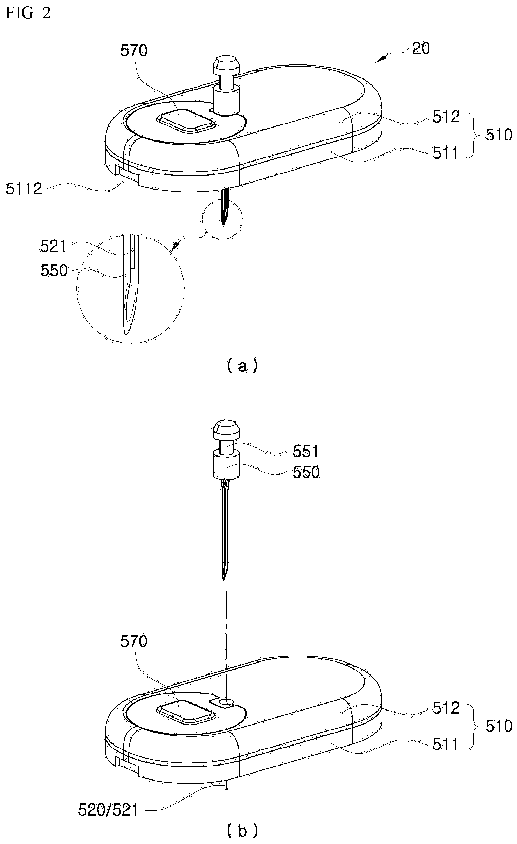

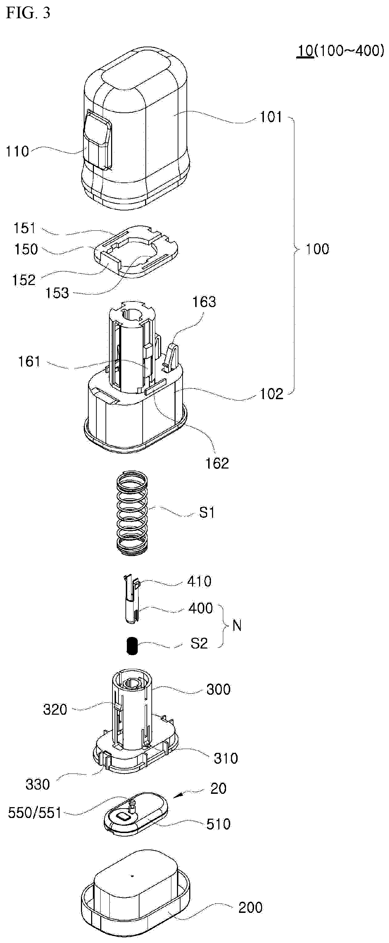

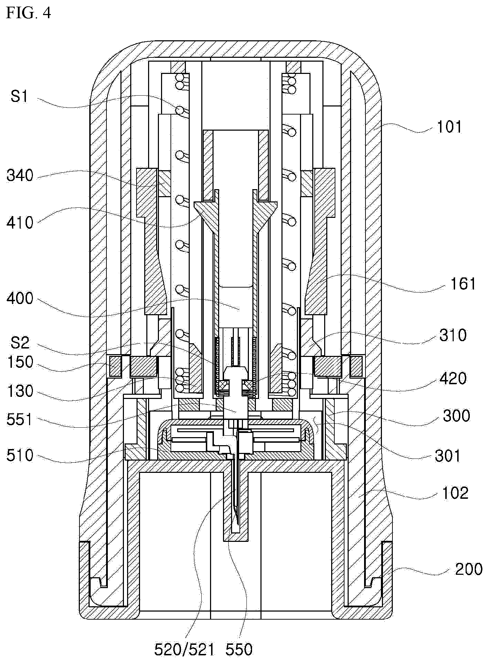

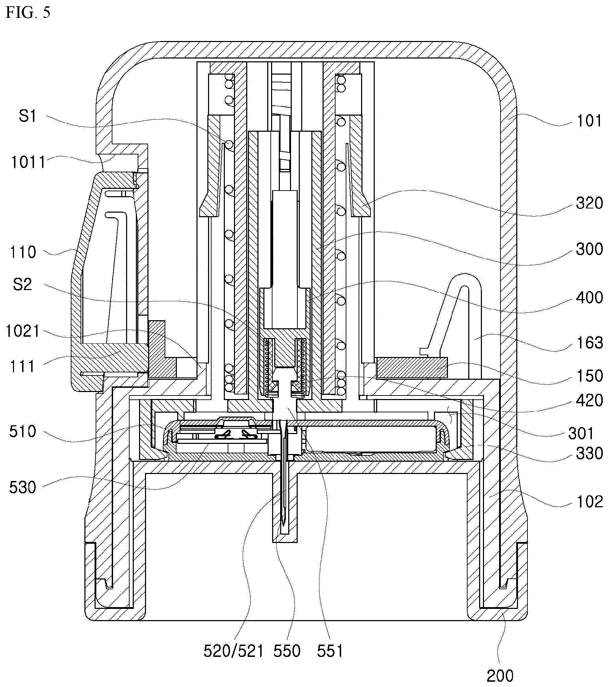

FIG. 1 is a perspective diagram illustrating an outline of an outward shape of a continuous biometric information measurement device according to an example embodiment of the present disclosure. FIG. 2 is a perspective diagram illustrating an outline of an outward shape of a body-attachable unit according to the present disclosure. FIG. 3 is an exploded perspective diagram illustrating an outline of a configuration of the continuous biometric information measurement device according to an example embodiment of the present disclosure. FIG. 4 is a cross-sectional diagram taken along line B-B of FIG. 1. FIG. 5 is a cross-sectional diagram taken along line A-A of FIG. 1.

A body-attachable unit 20 is assembled in an applicator 10, so that the continuous biometric information measurement device according to an example embodiment of the present disclosure is manufactured as one unit product. The continuous biometric information measurement device according to an example embodiment of the present disclosure has a shape in which an additional task of a user is minimized when a sensor applicator assembly is used and also has a structure of which a use scheme is greatly simple. Hereinafter, blood sugar will be described as an example of biometric information, but a variety of biometric information in a field to which the present disclosure is applied may be measured.

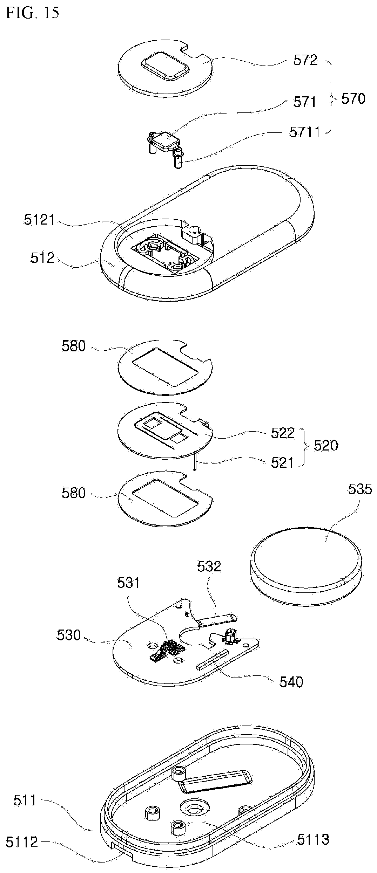

The body-attachable unit 20 is formed to be attachable to a body so as to periodically measure blood sugar from a body fluid and is formed so as to transmit a blood sugar measurement result to an external device such as an external terminal (not illustrated). Since a sensor member 520 of which an end portion is inserted into the body and a wireless communication chip 530 (of FIG. 15) for wireless communication with the external terminal are disposed in the body-attachable unit 20, the body-attachable unit 20 may be used without a need to additionally couple an additional transmitter thereto.

The applicator 10 is formed so that the body-attachable unit 20 is fixed and coupled to an inside thereof and operated by manipulation by a user so as to discharge the body-attachable unit 20 to an outside.

At this point, the body-attachable unit 20 is assembled and manufactured in a state of being inserted into the applicator 10 and is configured so as to move in a direction of outward discharge according to operation of the applicator 10 by the manipulation by the user and be attached to the body.

In other words, since the body-attachable unit 20 is assembled and manufactured so that the body-attachable unit 20 is attached to skin with only the operation of the applicator 10 in the state of being inserted into the applicator 10 in manufacture of a continuous glucose monitoring system according to an example embodiment of the present disclosure, and since the continuous glucose monitoring system is provided to the user in such a state, the user may attach the body-attachable unit 20 to the skin with only a task of simply operating the applicator 10 without an additional task for attaching the body-attachable unit 20 to the skin. Particularly, since an additional wireless communication chip is provided to the body-attachable unit 20, coupling the additional transmitter thereto is not required, and accordingly, the continuous glucose monitoring system may be further conveniently used.

A body-attachable unit of a general continuous glucose monitoring system in the related art is accurately inserted into an applicator after packaging of the body-attachable unit which is separately packaged is removed, and then the applicator is operated, so that the body-attachable unit is attached to the skin. However, a task of accurately inserting the body-attachable unit into the applicator is complicate and difficult. Also, glucose monitoring accuracy is decreased for a reason that a child or an elderly person contaminates the body-attachable unit when performing such a task or the like.

The continuous glucose monitoring system according to an example embodiment of the present disclosure is manufactured and distributed in a state in which the body-attachable unit 20 is inserted into the applicator 10 in the manufacture, and through this, a process in which the user unpacks the body-attachable unit 20 and inserts the body-attachable unit 20 into the applicator 10 is omitted. The body-attachable unit 20 may be attached to the skin with only simple manipulation of the applicator 10. Thus, usability is greatly increased, and particularly, since contamination or the like of the body-attachable unit 20 may be prevented, the glucose monitoring accuracy may be improved.

As such, since the glucose monitoring system according to an example embodiment of the present disclosure is manufactured in the state in which the body-attachable unit 20 is inserted into the applicator 10, it is desired that the body-attachable unit 20 and the applicator 10 are used in a disposable manner, not a reusable manner. For such a non-reusable structure, the applicator 10 according to an example embodiment is formed so that reinsertion of the body-attachable unit 20 is not allowed after the body-attachable unit 20 which is inserted therein operates one time so as to be discharged outward.

That is, the applicator 10 is formed in a shape of which a surface is open, and the body-attachable unit 20 is discharged outward through the open surface of the applicator 10. The applicator 10 may be configured not to allow insertion of another body-attachable unit 20 by the user so that the other body-attachable unit 20 is not inserted into the applicator 10 and not used after the body-attachable unit 20 therein is discharged outward through earliest one-time operation of the applicator 10.

Meanwhile, an additional protective cap 200 may be coupled to the applicator 10 in a separable manner so that exposure of the body-attachable unit 20 to the outside is blocked in a state of being inserted into the applicator 10. The applicator 10 may be configured so that the user attaches the body-attachable unit 20 to the body by operating the applicator 10 only after separating the protective cap 200.

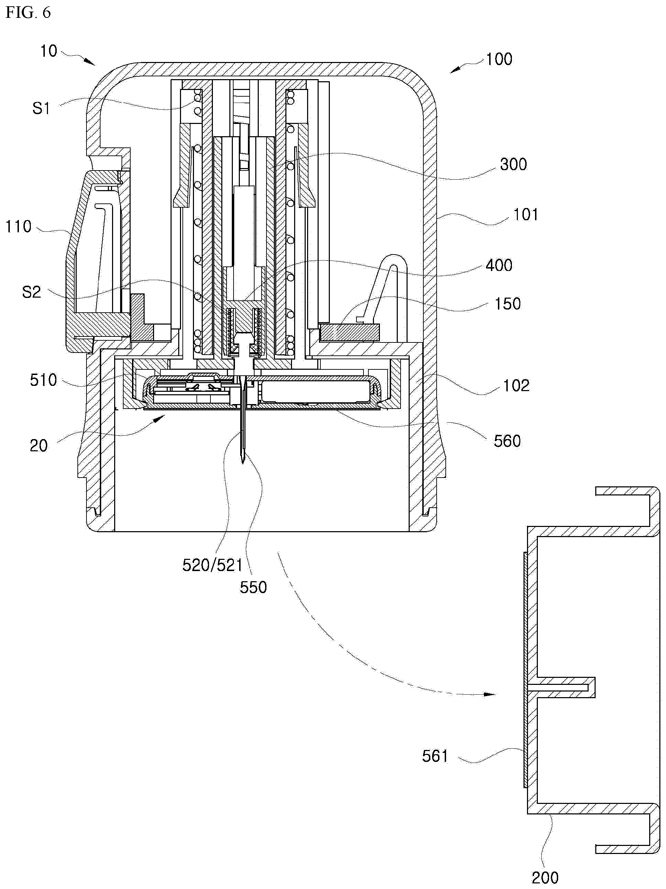

At this point, adhesive tape 560 (of FIG. 6) is attached to a body contact surface of the body-attachable unit 20 so that the body-attachable unit 20 may be attached to the body, and a release paper 561 (of FIG. 6) is attached to a body contact surface of the adhesive tape 560 for protection of the adhesive tape 560. The release paper(561) of the adhesive tape(560) may be formed so as to be separated and removed from the adhesive tape 560 in a process of separating the protective cap 200 from the applicator 10.

For example, the release paper 561 may be configured so that a side thereof is bonded to the protective cap 200, and thus, may be separated together with the protective cap 200 from the adhesive tape 560 when the user separates the protective cap 200 from the applicator 10. When the user separates the protective cap 200 accordingly, since the release paper 561 of the adhesive tape 560 is separated and removed, the body-attachable unit 20 may be attached to the body by operating the applicator 10 in such a state.

In addition, the applicator 10 may be formed so as to couple and fix the body-attachable unit 20 thereto in a state in which the body-attachable unit 20 is inserted at first a position therein and decouple and unfix the body-attachable unit 20 therefrom at a second position to which the body-attachable unit 20 moves to be discharged outward. Thus, since the body-attachable unit 20 is maintained to be in a fixed state in a state in which the body-attachable unit 20 is inserted and assembled in the applicator 10, and since the applicator 10 and the body-attachable unit 20 are decoupled and unfixed from each other when the body-attachable unit 20 is discharged outward by operating the applicator 10 and configured to be attached to the skin, the applicator 10 is separated from the body-attachable unit 20 when being separated in such a state, and only the body-attachable unit 20 remains in a state of being attached to the skin.

Meanwhile, the body-attachable unit 20 according to an example embodiment of the present disclosure may be formed so that the body-attachable unit 20 starts to operate through an additional switch device manipulated by the user. That is, after the body-attachable unit 20 is inserted into and attached to the body through the applicator 10, the user may allow the body-attachable unit 20 to start to operate through a switch device or the like provided to the body-attachable unit 20, and the body-attachable unit 20 may measure the blood sugar in the body from a such operation start time point and transmit the measurement result to the external terminal. At this point, the switch device manipulated by the user may be configured in various ways. Such a switch device and the body-attachable unit 20 will be described in detail below.

Also, in the body-attachable unit 20, the sensor member 520 is disposed in a housing 510 that is formed and separated into an upper housing 512 and a lower housing 511, and the end portion of the sensor member 520 is formed so as to protrude outward from the housing 510 to be inserted into and attached to the body. The sensor member 520 is formed of a sensor probe part 521 inserted into the body and a sensor body part 522 (of FIG. 15) disposed in the housing 510. The sensor probe part 521 and the sensor body part 522 may form the end portion and another end portion of the sensor member 520, respectively, in a bent form.

At this point, in order to smoothly perform a process of inserting the sensor member 520 into the body, an additional needle part 550 may be coupled to the housing 510 in a separable manner. The needle part 550 surrounds the end portion of the sensor member 520 so that the end portion of the sensor member 520 is stably inserted into the body and is formed so as to be inserted together with the sensor member 520 into the body.

The needle part 550 is mounted in a separable manner in a direction vertically penetrating the housing 510 of the body-attachable unit 20 as illustrated in FIG. 2 and formed in a shape surrounding an exterior of the sensor 520. A needle head 551 is formed at an upper end portion of the needle part 550. When the body-attachable unit 20 is moved by the applicator 10 in the direction of the outward discharge, the needle part 550 is inserted into the body prior to the sensor member 520 and assists the sensor member 520 to be stably inserted into the skin. The needle part 550 is coupled to a needle withdrawing body 400 of the applicator 10 through the needle head 551 and formed so as to be withdrawn and removed from the body by the needle withdrawing body 400 of the applicator 10 after the body-attachable unit 20 is inserted into and attached to the body by the operation of the applicator 10.

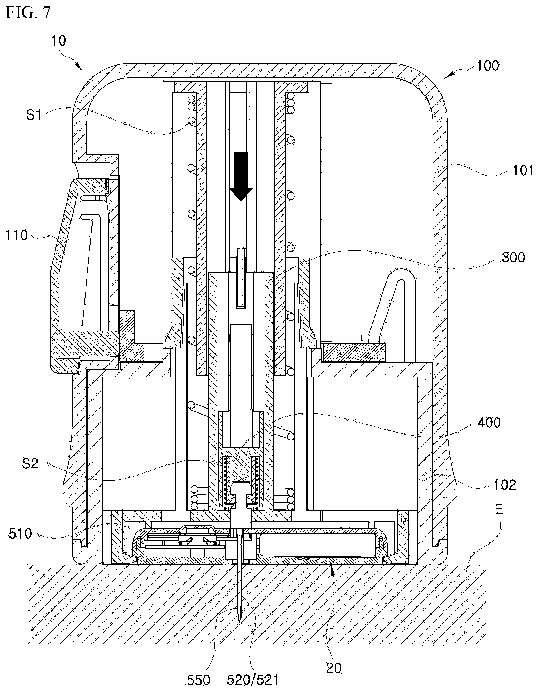

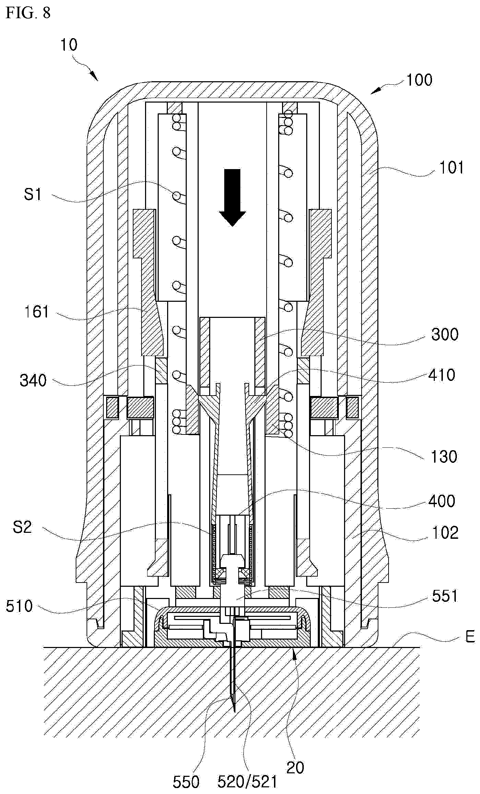

Then a state of using the above-described sensor applicator assembly will be described by focusing on FIGS. 6 through 10.

FIGS. 6 through 10 are diagrams illustrating states of using an applicator assembly according to an example embodiment of the present disclosure by operation according to an operation order.

To begin with, the protective cap 200 of the applicator 10 is separated as illustrated in FIG. 6. In a process of separating the protective cap 200, the release paper 561 of the adhesive tape 560 of the body-attachable unit 20 is separated together with the protective cap 200 to be removed from the adhesive tape 560. Afterward, a sensor applicator assembly is positioned at a bodily position at which the body-attachable unit 20 may be attached. In such a state, after a pressing button 110 is switched from a safe mode to a state of a pressing waiting mode, the pressing button 110 is manipulated and pressed. Here, the safe mode is a state in which the pressing button 110 is caught on a step of a housing(510) so as not to be pushed, and the pressing waiting mode is a state in which the pressing button 110 is pushed upward to be released from the step of the housing(510) so that a shooting plate may be pushed.

When the pressing button 110 is manipulated and pressed, since a shooting plate 150 moves to disengage from a plunger body 300, the plunger body 300 is moved downward by a plunger elastic spring S1 in a direction of outward discharge. The body-attachable unit 20 is fastened to the plunger body 300 and disposed at a lower end of the plunger body 300. When the plunger body 300 moves downward, the body-attachable unit 20 moves downward together with the plunger body 300 from a first position to a second position in the direction of the outward discharge.

In this process, the needle part 550 and the sensor probe part 521 of the body-attachable unit 20 is inserted into a body E. At this point, the body-attachable unit 20 may be bonded to a surface of the body E by the adhesive tape 560 of a bottom surface. As such, when the plunger body 300 moves in the direction of the outward discharge, the plunger body 300 may engage with a return prevention hook 161 of an inner case 102 not to move back upward. Thus, the applicator 10 which is used once may not be reused again.

Meanwhile, the plunger body 300 is formed so that a lower end surface of the plunger body 300 and the body-attachable unit 20 which is coupled to the plunger body 300 has a height equal to that of a lower end surface of a main case 100 in a state in which the plunger body 300 moves to the second position in the direction of the outward discharge. However, a lower end surface of a plunger body A300 may be formed so as to protrude downward further than a lower end surface of a main case A100 by a distance of X. Accordingly, the lower end surface of the body-attachable unit 20 which is coupled to the plunger 300 also protrudes downward further than the lower end surface of the main case 100 by the distance of X.

As such, the plunger body 300 may be formed so as to protrude further from an opening of the main case 100 when the plunger body 300 elastically moves, and through this, the body-attachable unit 20 which is coupled to the plunger body 300 may be further strongly attached to a body surface. Particularly, even when a user slightly lifts or move the main case 100 from the body surface out of fear in a process of manipulating the applicator(10), since the plunger body 300 operates to protrude further than the opening of the main case 100, the body-attachable unit 20 may be stably pressed on and make contact with the body surface.

When the plunger body 300 moves downward, a sensor fixation hook 330 of a sensor receiving part 301 may disengage from a body-attachable unit A20. In addition, an elastic hook 410 of the needle withdrawing body 400 is pressed inward by a needle withdrawing pressing part 130 of the inner case 102 to disengage from the plunger body 300.

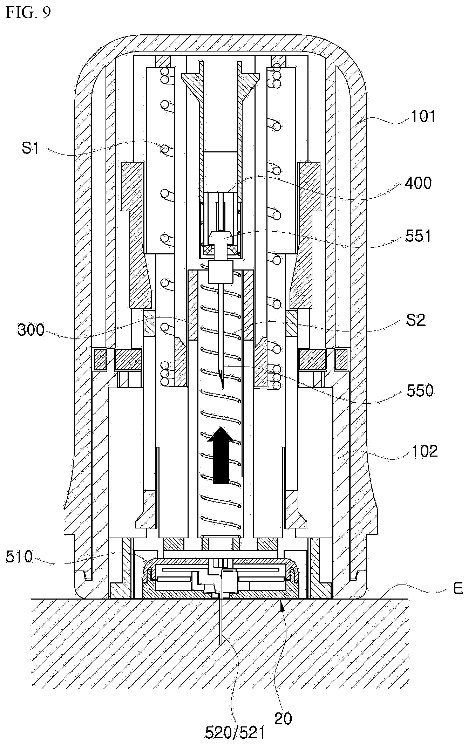

Thus, as illustrated in FIG. 9, when the plunger body 300 moves downward, at the same time, the needle withdrawing body 400 is moved and returned upward by the needle withdrawing elastic spring S2. At this point, since the needle part 550 moves upward together with the needle withdrawing body 400, the needle part 550 is withdrawn and removed from the body E.

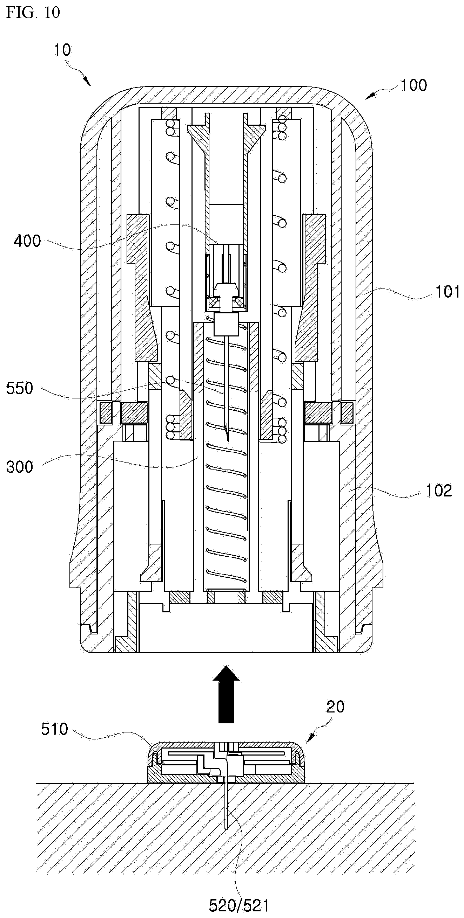

In this state, as described above, since the sensor fixation hook 330 and the body-attachable unit 20 are to disengage from each other, the applicator 10 may be separated upward and removed as illustrated in FIG. 10. When the applicator 10 is separated and removed as such, only the body-attachable unit 20 is in a state of being attached to the body E.

Afterward, a pressing operation module 570 or the like of the body-attachable unit 20 is manipulated, so that the body-attachable unit 20 may starts to operate. Accordingly, a blood sugar measurement result by the body-attachable unit 20 is transmitted to an additional external terminal.

A scheme in which after the body-attachable unit 20 is discharged outward to the second position together with the plunger body 300 and inserted into the body E, the needle withdrawing body 400 moves and returns upward to withdraw and remove the needle part 550 from the body E has been described above. In contrast, a needle withdrawing device N may be configured in a scheme in which before a sensor member A520 of the body-attachable unit A20 is inserted into the body, a needle part A550 is withdrawn and removed from the body E.

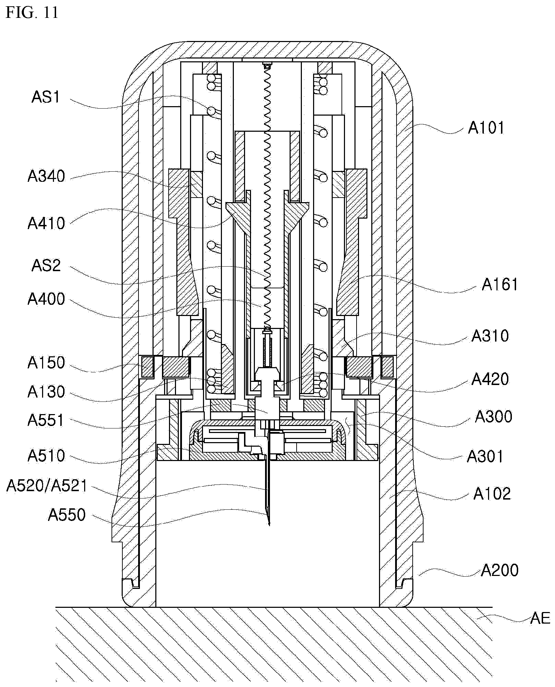

Meanwhile, the needle withdrawing elastic spring S2 for moving and returning the needle withdrawing body 400 to the first position in the main case 100 has been described above as having a form of a compression spring, but may be formed in a shape of a tension spring as illustrated in FIGS. 11 through 13.

Both ends of the needle withdrawing elastic spring S2 which has the form of the compression spring are coupled to the plunger body 300 and the needle withdrawing body 400, and the needle withdrawing elastic spring S2 having the form of the compression spring is configured in a scheme in which the needle withdrawing elastic spring S2 is disposed in a compressed form to move and return the needle withdrawing body 400 toward the first position with a tension restoration elastic force.

As illustrated in FIGS. 11 through 13, the both ends of the needle withdrawing elastic spring S2 which has a form of the tension spring are coupled to the main case 100 and the needle withdrawing body 400, and the the needle withdrawing elastic spring S2 having the form of the tension spring is extended as the needle withdrawing body 400 moves downward toward the second position together with the plunger body 300 and configured to apply an elastic force toward the first position to the needle withdrawing body 400 with a compression restoration force of the extended spring.

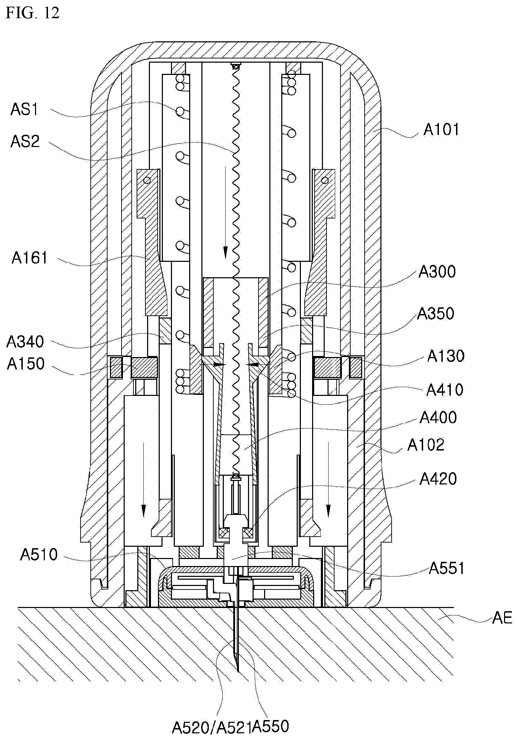

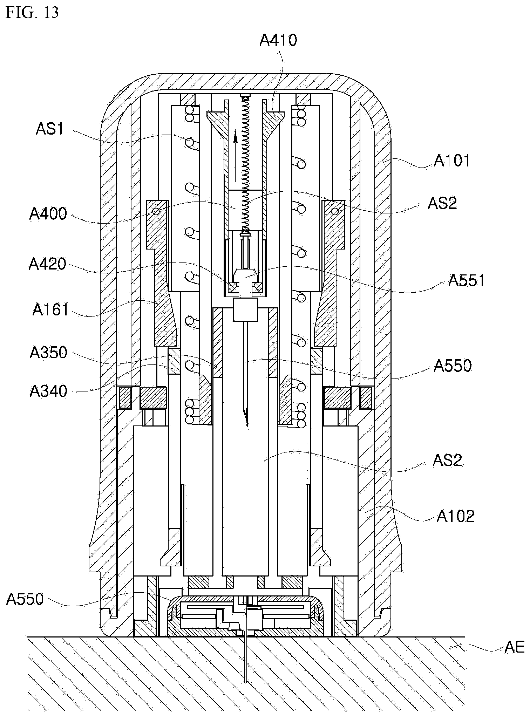

Thus, when the plunger body 300 and the body-attachable unit 20 are discharged outward from an inside of the main case 100 to the second position as shown to be switched from a state illustrated in FIG. 11 to a state illustrated in FIG. 12, the needle withdrawing elastic spring S2 is extended to generate the compression restoration force. The elastic force toward the first position is applied to the needle withdrawing body 400 by the compression restoration force. At this point, since the elastic hook 410 of the needle withdrawing body 400 is pressed by the needle withdrawing pressing part 130 of the inner case 102 to disengage from the plunger body 300, the needle withdrawing body 400 is moved upward toward the first position by the compression restoration force of the needle withdrawing elastic spring S2 as illustrated in FIG. 13. In this case, the needle part 550 moves with movement of the needle withdrawing body 400 to be withdrawn and removed from the body E.

The needle withdrawing elastic spring S2 which has a form of such a tension spring may be applied to a structure illustrated in FIGS. 1 and 2.

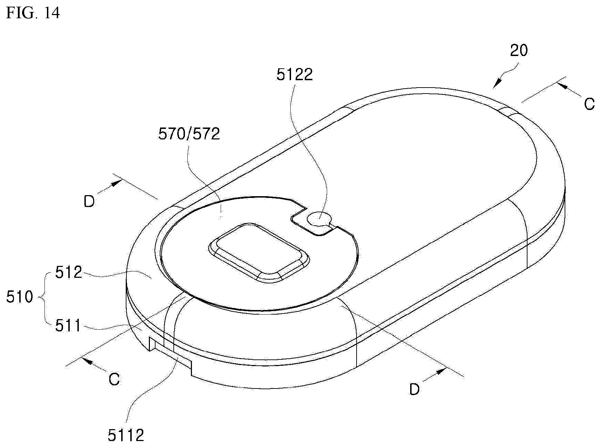

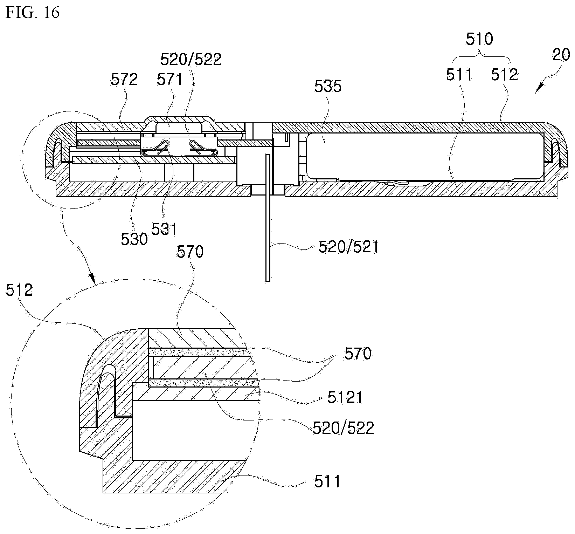

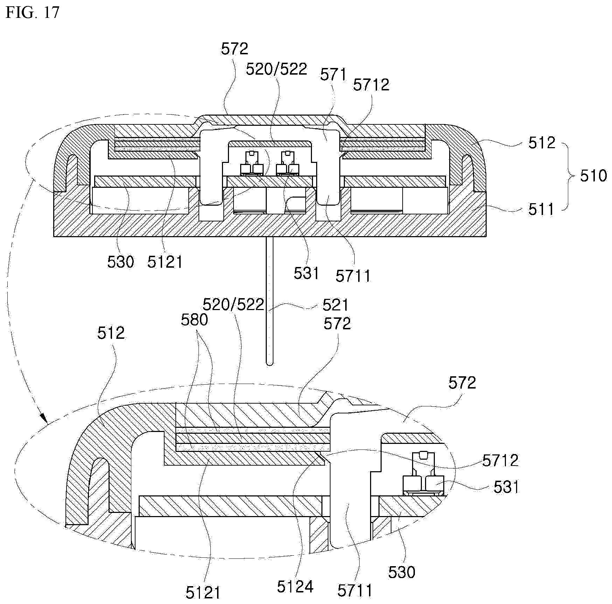

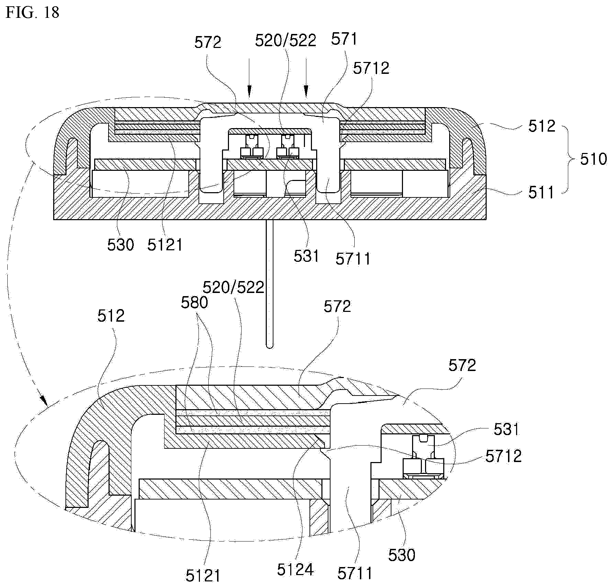

FIG. 14 is a perspective diagram illustrating an outline of an outward shape of a body-attachable unit attached to a body according to an example embodiment of the present disclosure. FIG. 15 is an exploded perspective diagram illustrating an outline of a configuration of the body-attachable unit according to an example embodiment of the present disclosure. FIG. 16 is a cross-sectional diagram taken along line C-C of FIG. 14. FIG. 17 is a cross-sectional diagram taken along line D-D of FIG. 14. FIG. 18 is a diagram illustrating an outline of an operation state of a pressing operation module according to an example embodiment of the present disclosure.

The body-attachable unit 20 according to an example embodiment of the present disclosure includes and is formed of the housing 510 to which adhesive tape is attached so that a bottom surface is attached to skin, the sensor member 520 of which an end portion is disposed in the housing 510 so as to protrude outward from the bottom surface of the housing 510 and the end portion is inserted into the body if the housing 510 is attached to the skin, and a printed circuit board (PCB) substrate 530 disposed in the housing 510.

The sensor member 520 is formed so that the end portion is inserted into the body and another end portion makes contact with the PCB substrate 530. The sensor body part 522 is formed at the other end portion so as to make contact with an electrical contact of the PCB substrate 530. The sensor probe part 521 which is extended and formed in a shape bent from a side of the sensor body part 522 and protrudes outward from the housing 510 to be inserted into the body is formed at the end portion. The sensor body part 522 is formed in a shape having a relatively wide area. The sensor probe part 521 is formed in a relatively narrow and long shape.

The housing 510 may be formed and separated into the upper housing 512 and the lower housing 511, so that an inner receiving space is formed. In the housing 510, a sensor support part 5121 supporting the sensor body part 522 so that the sensor body part 522 is spaced apart from an electrical contact 531 of the PCB substrate 530 by a predetermined interval is formed, and a sensor guide part (not illustrated) for supporting and guiding some sections of the probe part 521 is also formed. Also, a substrate support part 5113 for fixing and supporting the PCB substrate 530 at a predetermined position may be formed in the housing 510.

The electrical point 531 is formed to the PCB substrate 530 so as to be electrically connected to the sensor member 520. A wireless communication chip 540 is mounted to the PCB substrate 530 so as to transmit a blood sugar measurement result measured through the sensor member 520 to an external terminal. In an example embodiment of the present disclosure, as the wireless communication chip 540 is provided in the body-attachable unit 20 as such, communication with the external terminal may be easily performed without a task of connecting an additional transmitter.

In addition, a battery 535 is mounted in the housing 510 so as to supply power to the PCB substrate 530. At this point, the battery 535 is disposed in an area independent from the PCB substrate 530, not disposed in a form mounted on a surface of the PCB substrate 530. In other words, areas of the PCB substrate 530 and the battery 535, which are projected on the bottom surface of the housing 510, are independently disposed without an overlapping area. As such, the PCB substrate 530 and the battery 535 are disposed in areas independent from each other. Through this, the body-attachable unit 20 may have a decreased thickness and may be further minimized. At this point, an additional contact terminal 532 may be formed to the PCB substrate 530 and extended toward the battery 535 so as to make electrical contact with and connected to the battery 535.

The body-attachable unit 20 according to an example embodiment of the present disclosure is formed so that the other end portion of the sensor member 520, namely, the sensor body part 522 makes contact with the electrical contact 531 of the PCB substrate 530 by manipulation by a user. The body-attachable unit 20 is configured to start to operate with such electrical contact. That is, the wireless communication chip 540 or the like may be configured to start to operate while the power is supplied by electrical connection between the sensor member 520 and the PCB substrate 530 by the manipulation by the user.

In order to allow the other end portion of the sensor member 520 and the electrical contact 531 of the PCB substrate 530 to be in contact by the manipulation of the user, the additional pressing operation module 570 which is operated by manipulation by the user may be provided.

The pressing operation module 570 may include a moving pressing body 571 movably coupled to the housing 510 and moved by a pressing force by the user in a direction of pressing and may be formed so that with movement of the moving pressing body 571, at least some areas of the other end portion of the sensor member 520 is pressed and transformed by the moving pressing body 571 to make contact with the electrical contact 531 of the PCB substrate 530.

Also, the pressing operation module 570 may further include a button cover 572 having a soft material, which is coupled to the housing 510 to be exposed outward in a form surrounding an outer space of the moving pressing body 571 so that pressing manipulation by the user is allowed. A coupling area of the button cover 572 and the housing 510 may be configured to be processed to be sealed.

At this point, a scheme of a sealing process for the coupling area of the button cover 572 and the housing 510 may be formed of a scheme using double-sided tape 580. For example, the double-sided tape 580 may be bonded to a surface of the sensor body part 522, namely, the other end portion of the sensor member 520, along an edge circumference thereof. An inner side surface of the button cover 572 may be bonded, along an edge circumference thereof, to another surface of the double-sided tape 580. The edge circumference of the button cover 572 may be processed to be sealed by the double-sided tape 580. In this case, the double-sided tape 580 may be also bonded to another surface of the sensor body part 522 along the edge circumference thereof. Through this, the edge circumference of the sensor body part 522 may be bonded and fixed to the sensor support part 5121 with the double-sided tape 580.

In a state in which the edge circumference of the sensor body part 522 is bonded and fixed to the sensor support part 5121 through the double-sided tape 580 as such, as illustrated in FIG. 18, a central area of the sensor body part 522 is pressed and transformed by the moving pressing body 571 to make contact with the electrical contact 531 of the PCB substrate 530. The moving pressing body 571 moves in the direction of the pressing. However, since the button cover 572 has the soft material, and since an edge portion of the button cover 572 is bonded to the housing 510 by the double-sided tape 580, only a central area thereof is transformed in the direction of the pressing, and the edge portion is bonded and fixed, so that a sealing state is maintained.

Meanwhile, after the sensor body part 522 makes contact with the electrical contact 531 of the PCB substrate 530 by the manipulation by the user, it is desired that a contact state is stably maintained for stable measurement of blood sugar. To this end, the moving pressing body 571 may be formed so that a position thereof is fixed in a state in which the moving pressing body 571 is moved by the pressing force by the user in the direction of the pressing.

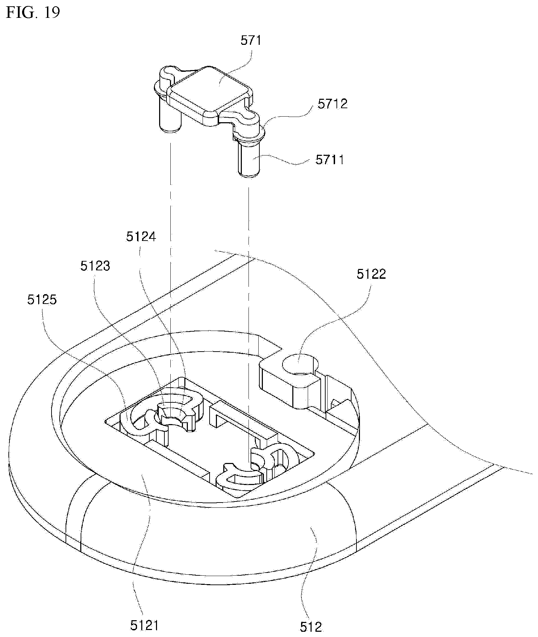

**In** order to fix the position of the moving pressing body 571 as such, as illustrated in FIG. 19, a protrusion guide part 5711 protruding in a direction of the movement of the moving pressing body 571 may be formed to the moving pressing body 571, and a catch hook 5712 may be formed on an outer circumferential surface of the protrusion guide part 5711. **In** addition, an engagement protrusion 5124 which the catch hook 5712 of the protrusion guide part 5711 may engage with and be coupled to in a state in which the moving pressing body 571 moves in the direction of the pressing may be formed to the housing 510. As illustrated in FIG. 19, the moving pressing body 571 may be configured so that the position thereof is fixed as the catch hook 5712 engages with and is coupled to the engagement protrusion 5124.

At this point, the engagement protrusion 5124 may be formed to the sensor support part 5121 of the housing 510. As illustrated in FIG. 19, at least two guide fixation parts 5123 having a form surrounding the protrusion guide part 5711 of the moving pressing body 571 may be formed to be spaced apart from each other in a circumferential direction, and the engagement protrusion 5124 may be formed to each guide fixation part 5123. Also, each guide fixation part 5123 may be disposed in a form elastically supported by an elastically transformable elastic support part 5125.

Thus, in a process in which the moving pressing body 571 moves in the direction of the pressing, the guide fixation part 5123 facilitates the movement of the moving pressing body 571 while being elastically transformed. When the movement of the moving pressing body 571 is completed, the guide fixation part 5123 is elastically returned, and the catch hook 5712 engages with and is coupled to the engagement protrusion 5124. Since the guide fixation part 5123 is elastically supported by the elastic support part 5125, a state in which the catch hook 5712 and the engagement protrusion 5124 engage and are coupled is stably maintained.

Meanwhile, the sensor member 520 is formed of the sensor body part 522 and the sensor probe part 521 as described above. A pressed transformation part 523 that is transformed by pressing movement of the moving pressing body 571 to make contact with the electrical contact 531 is formed to the sensor body part 522.

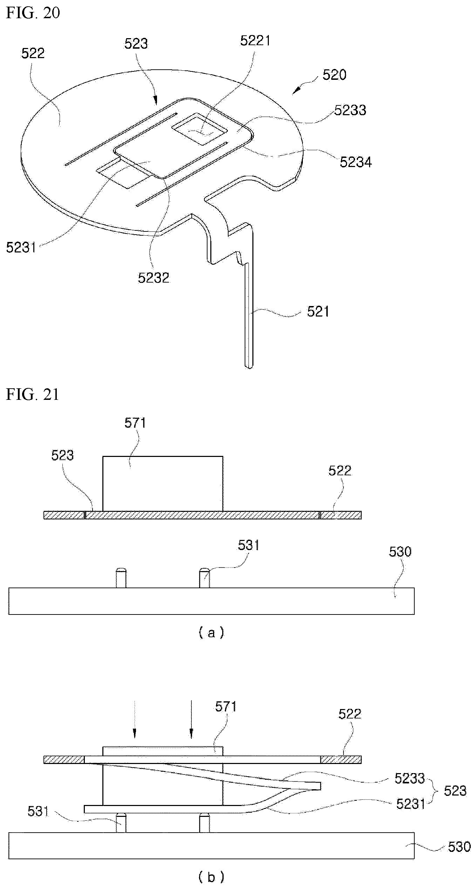

As illustrated in FIG. 20, the pressed transformation part 523 may include a first cut area 5231 having a form cut along a first cut line 5232 formed in the central area of the sensor body part 522 and may be formed so that the first cut area 5231 is pressed and transformed by the moving pressing body 571.

In addition, the pressed transformation part 523 may include a second cut area 5233 having a form cut along a second cut line 5234 formed in an outer area of the first cut line 5232 in the central area of the sensor body part 522 and may be formed so that the first cut area 5231 and the second cut area 5233 are pressed and transformed by the moving pressing body 571.

At this point, the first cut line 5232 is formed in a shape in which some sections of a closed loop is open, and the second cut line 5234 is formed so as to have an open section at a position opposite to an open section of the first cut line 5232 in a shape of a closed loop surrounding the open section of the first cut line 5232 at an outside.

When the moving pressing body 571 is manipulated and pressed according to such a structure, as illustrated in (a) and (b) of FIG. 21, the first cut area 5231 of the pressed transformation part 523 is elastically transformed downward, and the second cut area 5233 which is formed in an outer area of the first cut area 5231 is successively and sequentially elastically transformed downward. Accordingly, since the first cut area 5231, which makes direct contact with the electrical contact 531 of the PCB substrate 530, relatively horizontally makes contact with the electrical contact 531 of the PCB substrate 530, a state in which the sensor body part 522 makes contact with the electrical contact 531 may be further stably maintained.

Meanwhile, a plurality of electrical contacts 531 that make electrical contact with the sensor body part 522 may be formed to the PCB substrate 530 in a shape protruding toward the sensor body part 522. At least one of the plurality of electrical contacts 531 may be formed to have a protrusion height higher than those of remaining of the plurality of electrical contacts 531.

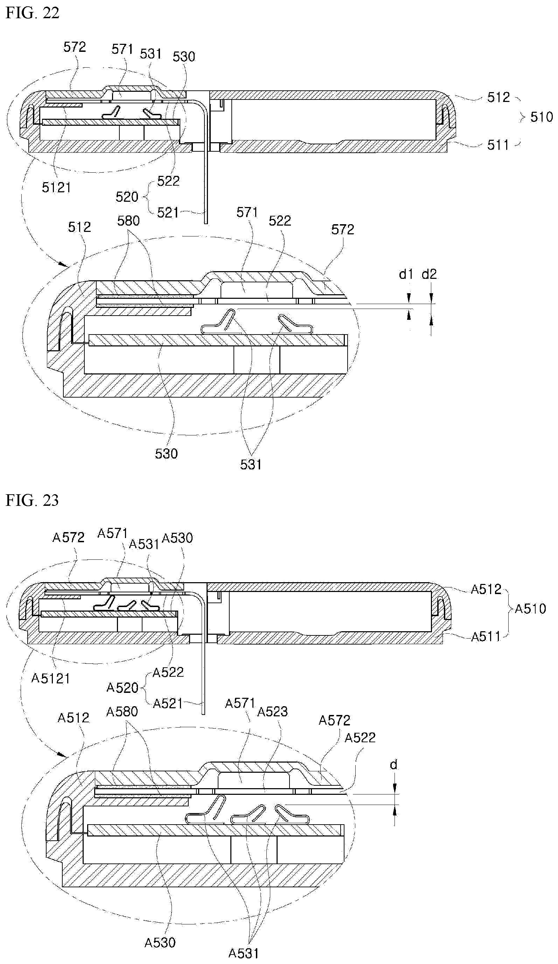

For example, when two electrical contacts 531 are formed to the PCB substrate 530 as illustrated in FIG. 22, one electrical contact 531 is formed to have a protrusion height higher than a protrusion height of another electrical contact 531. Accordingly, intervals at which the sensor body part 522 is spaced apart therefrom are differently formed as d1 and d2.

Through such a disposition structure, the sensor body part 522 may be prevented from making contact with the electrical contact 531 without the pressing manipulation by the user for a reason of a manufacturing and assembly tolerance or the like.

However, forming the at least one of the plurality of electrical contacts 531 to have the protrusion height higher than those of the remaining of the plurality of electrical contacts 531 and, at the same time, disposing and spacing, from the sensor body part 522, an electrical contact that is formed and protrudes higher requires a greatly precise process.

A disposition relationship between a sensor member and an electrical contact according to another example embodiment of the present disclosure will be described.

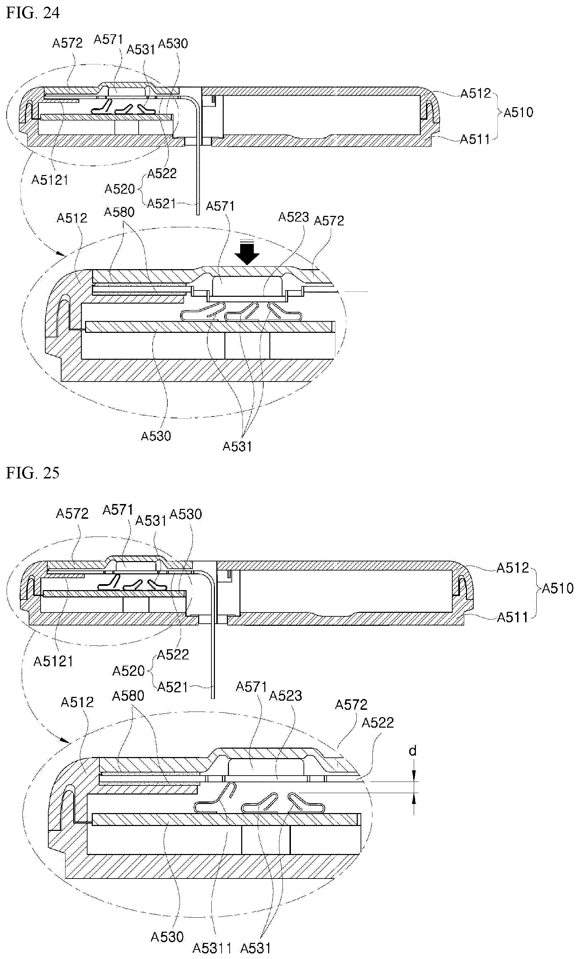

FIG. 23 is a diagram conceptually illustrating a disposition relationship between a sensor member and an electrical contact according to another example embodiment of the present disclosure. FIG. 24 is a diagram conceptually illustrating a state in which the sensor member and the electrical contact illustrated in FIG. 23 are in contact.

In the disposition relationship between the sensor member and the electrical contact according to another example embodiment of the present disclosure, the body-attachable unit A20 includes and is formed of a housing A510 of which a bottom surface is attached to skin as described above, a PCB substrate A530, which is disposed in the housing A510, with a plurality of electrical contacts A531 formed to protrude from a surface thereof, and the sensor member A520 including a sensor body part A522 and a sensor probe part A521. A detailed configuration of the body-attachable unit A20 is identical to that described above. Hereinafter, a configuration different from the above-described configuration will be mainly described in order to prevent redundant descriptions.

The sensor body part A522 of the sensor member A520 is formed so as to be pressed and transformed by manipulation by a user to make contact with an electrical contact A531 of the PCB substrate A530.

A pressing operation module A570 operated by manipulation by the user is coupled to the housing A510. The pressing operation module A570 is configured so as to press the sensor body part A522 by the manipulation by the user so that the sensor body part A522 makes contact with all of the plurality of electrical contacts A531. The pressing operation module A570 may include a moving pressing body A571 moved by a pressing force by the user in a direction of pressing. The pressed transformation part A523 transformed by pressing of the the moving pressing body A571 to make contact with the electrical contact A531 of the PCB substrate A530 is formed to the sensor body part A522.

Thus, as the user operates the pressing operation module A570, the pressed transformation part A523 of the sensor body part A522 may be transformed to make contact with all of the plurality of electrical contacts A531 simultaneously. In a structure of an electrical contact of the body-attachable unit according to another example embodiment of the present disclosure, the sensor body part A522 may make contact with all of the plurality of electrical contacts A531 simultaneously, and through this, the body-attachable unit A20 may be configured to start to operate.

In a disposition structure of the electrical contact of the body-attachable unit according to another example embodiment of the present disclosure, as illustrated in FIG. 23, at least one of the plurality of electrical contacts A531 may make direct contact with the sensor body part A522, and remaining of the plurality of electrical contacts A531 may be formed to be spaced apart from the sensor body part A522.

For example, one electrical contact A531 among the plurality of electrical contacts A531 of the PCB substrate A530 may make direct contact with the sensor body part A522 to support the sensor body part A522, and remaining of the plurality of electrical contacts A531 may be configured to be spaced apart from the sensor body part A522.

The plurality of electrical contacts A531 may be formed to protrude upward from an upper surface of the PCB substrate A530 and formed so as to be elastically transformable downward when pressed by the sensor body part A522. At this point, the electrical contact A531 that makes contact with the sensor body part A522 may be formed to have a protrusion height higher than those of the remaining of the plurality of electrical contacts A531 to support the sensor body part A522.

One of the plurality of electrical contacts A531 may make contact with the sensor body part A522 to support the sensor body part A522 as such, and through this, the sensor body part A522 may be prevented from making contact with all of the plurality of electrical contacts A531 simultaneously due to a manufacturing and assembly tolerance, a mistake in a task in a manufacturing process, or the like in manufacturing and assembly.

When described in further detail, since the housing A510 is formed in a greatly thin shape, stably maintaining therein a state in which the sensor body part A522 and the electrical contact A531 are spaced apart is greatly difficult. Particularly, a possibility of a defect that leads contact of the sensor body part A522 with all of the plurality of electrical contacts A531 due to an assembly tolerance, the mistake in the task in the manufacturing process, or the like is greatly high. When the sensor body part A522 makes contact with all of the plurality of electrical contacts A531 simultaneously, since the body-attachable unit A20 starts to operate, a product error occurs in manufacturing and distribution.

As illustrated in FIG. 23, when one of the plurality of electrical contacts A531 makes contact with the sensor body part A522, the one electrical contact A431 supports the sensor body part A522. Accordingly, downward transformation of the sensor body part A522 is prevented, so that contact of the sensor body part A522 with another electrical contact A531 is prevented. That is, the sensor body part A522 is supported by allowing the one of the plurality of electrical contacts A531 to make contact with the sensor body part A522, and through this, contact of remaining of the plurality of electrical contacts A53 with the sensor body part A522 is prevented. Accordingly, since the sensor body part A522 doesn't make contact with all of the plurality of electrical contacts A531 simultaneously, a product defect may be reduced in the manufacturing and distribution. In addition, since a high degree of focusing on adjustment of the state in which the sensor body part A522 and the electrical contact A531 are spaced apart in the manufacturing process may be relieved, fatigue of a worker due to work may be reduced, and work convenience may be improved.

Meanwhile, the electrical contact A531 which supports the sensor body part A522 may be formed of an elastic material so as to be elastically transformed while maintaining a state of making contact with the sensor body part A522 as the sensor body part A522 is transformed by operation of the pressing operation module A570. Thus, as illustrated in FIG. 24, a state in which the sensor body part A522 and the electrical contact A531 which supports the sensor body part A522 are in contact may be stably maintained in a process in which the sensor body part A522 is transformed downward by the operation of the pressing operation module A570. In a state in which the downward transformation of the sensor body part A522 is completed, a state in which the sensor body part A522 and the plurality of electrical contacts A531 are mutually in contact may be also stably maintained.

Also, in the disposition relationship between the sensor member and the electrical contact according to another example embodiment, when only some electrical contacts A531 among the plurality of electrical contacts A531 make contact with the sensor body part A522, operation of the body-attachable unit A20 does not start. Such a operation start prevention function may be achieved through a simple scheme of configuring a pattern circuit of the PCB substrate A530 so that the operation starts only when all of the plurality of electrical contacts A531 makes contact with the sensor body part A522 or the like.

Meanwhile, in contrast, the electrical contact A531 which supports the sensor body part A522 is formed in a shape of a dummy contact not connected to the pattern circuit of the PCB substrate A530 and configured so as not to serve as an actual electrical contact.

In association therewith, as illustrated in FIG. 25, an additional sensor support member A5311 that makes contact with the sensor body part A522 and supports the body part A522 so that the body part A522 is spaced apart from the plurality of electrical contacts A531 may be formed to protrude from the PCB substrate A530.

The sensor support member A5311 may be formed in a configuration not connected to the pattern circuit of the PCB substrate A530 and in a shape similar to the electrical contact A531 which supports the sensor body part A522 illustrated in FIG. 23. In other words, the sensor support member A5311 may be formed to elastically protrude on the upper surface of the PCB substrate A530 in an elastically transformable shape and may be formed to have a protrusion height higher than those of the plurality of electrical contacts A531. Also, the sensor support member A5311 may be formed so as to be elastically transformed while maintaining a state of making contact with the sensor body part A522 as the sensor body part A522 is transformed by the operation of the pressing operation module A570.

According to such a structure, the sensor body part A522 may be supported by an elastic force by the sensor support member A5311 to maintain a state of being spaced apart from the plurality of electrical contacts A531 , and the sensor body part A522 is transformed by the operation of the pressing operation module A570 to make contact with all of the plurality of electrical contacts A531.

The above description is merely to describe an example of the technical idea of the present disclosure. Those skilled in the art to which the present disclosure belong may allow various changes and modification within a range not deviating from the essential characteristics of the present disclosure. Thus, example embodiments disclosed in the present disclosure is to describe the technical idea of the present disclosure, not to limit the technical idea, and the range of the technical idea of the present disclosure is not limited to those example embodiments. The scope of the present invention is defined by the following claims.

### LIST OF REFERENCE SIGNS

- 10: applicator
- 20: body-attachable unit
- 30: transmitter
- 100: main case
- 101: outer case
- 102: inner case
- 103: cover case
- 104: return prevention hook
- 110: pressing button
- 120: safety lock
- 130: pressing part
- 140: hook guide part
- 200: protective cap
- 300: plunger body
- 310: elastic hook
- 330: sensor fixation hook
- 400: needle withdrawing body
- 410: elastic hook
- 420: needle head holder
- S1: plunger elastic spring
- S2: needle withdrawing elastic spring
- 510: housing
- 520: sensor
- 530: PCB substrate
- 540: wireless communication chip
- 550: needle part
- 560: adhesive tape
- 561: release paper
- 531, A531: electrical contacts
- A5311: sensor support member

## Claims

1. A body-attachable unit (20, A20) configured to be attached to and inserted into a body for measuring biometric information, the body-attachable unit (20, A20) comprising:
a housing (510, A510) formed so that a bottom surface is configured to be attached to skin;
a printed circuit board, PCB, substrate (530, A530), which is disposed in the housing (510, A510), with a plurality of electrical contacts (531, A531) formed to protrude from a surface of the PCB substrate (530, A530); and
a sensor member (520, A520) comprising a sensor body part (522, A522) disposed in the housing (510, A510) and a sensor probe part (521, A521) formed and extended from a side of the sensor body part (522, A522) to protrude outward from the bottom surface of the housing (510, A510) and configured to be inserted into the skin if the housing (510, A510) is attached to the skin,
wherein at least one of the plurality of electrical contacts (531, A531) is formed to make contact with the sensor body part (522, A522) to support the sensor body part (522, A522),
wherein the remaining of the plurality of electrical contacts (531, A531) are formed to be spaced apart from the sensor body part (522, A522),
wherein the at least one of the plurality of electrical contacts (531, A531) supporting the sensor body part (522, A522) is not connected to a pattern circuit of the PCB substrate (530, A530), and
wherein the sensor body part (522, A522), in a state spaced apart from the remaining of the plurality of electrical contacts (531, A531), is configured to make contact with all of the plurality of electrical contacts (531, A531) by manipulation by a user.

2. The body-attachable unit (20, A20) of claim 1, further comprising a pressing operation module (570, A570) coupled to the housing (510, A510) to be operated by manipulation by the user and configured to press the sensor body part (522, A522) so that the sensor body part (522, A522) makes contact with all of the plurality of electrical contacts (531, A531).

3. The body-attachable unit (20, A20) of claim 2, wherein the sensor body part (522, A522) is configured to be transformed by pressing of the pressing operation module (570, A570) to make contact with all of the plurality of electrical contacts (531, A531).

4. The body-attachable unit (20, A20) of claim 3, wherein an electrical contact (531, A531) that makes contact with the sensor body part (522, A522) to support the sensor body part (522, A522) among the electrical contacts (531, A531) is configured to be elastically transformed while maintaining a state of making contact with the sensor body part (522, A522) as the sensor body part (522, A522) is transformed by the pressing operation module (570, A570).

5. The body-attachable unit (20, A20) of claim 1, wherein the electrical contacts (531, A531) are formed to elastically protrude from the PCB substrate (530, A530) in an elastically transformable shape.

6. The body-attachable unit (20, A20) of claim 5, wherein an electrical contact (531, A531) that makes contact with the sensor body part (522, A522) to support the sensor body part (522, A522) among the electrical contacts (531, A531) is formed to have a protrusion height from the PCB substrate (530, A530) higher than those of the remaining of the plurality of electrical contacts (531, A531).

## Patentansprüche

1. Am Körper anbringbare Einheit (20, A20), die dazu ausgebildet ist, an einem Körper angebracht und in diesen eingeführt zu werden, um biometrische Informationen zu messen, wobei die am Körper anbringbare Einheit (20, A20) aufweist:
ein Gehäuse (510, A510), das so ausgebildet ist, dass eine Bodenfläche an der Haut anbringbar ist;
ein Leiterplatten-Substrat (530, A530), kurz PCB-Substrat, das in dem Gehäuse (510, A510) angeordnet ist und mehrere elektrische Kontakte (531, A531) umfasst, die so ausgebildet sind, dass sie von einer Oberfläche des PCB-Substrats (530, A530) vorstehen; und
ein Sensorelement (520, A520), das ein Sensor-Körperteil (522, A522), das in dem Gehäuse (510, A510) angeordnet ist, und ein Sensor-Sondenteil (521, A521) aufweist, das so ausgebildet ist und sich von einer Seite des Sensor-Körperteils (522, A522) erstreckt, dass es von der Bodenfläche des Gehäuses (510, A510) nach außen vorsteht, und dazu ausgebildet ist, in die Haut eingeführt zu werden, wenn das Gehäuse (510, A510) an der Haut angebracht ist,
wobei mindestens einer der mehreren elektrischen Kontakte (531, A531) so ausgebildet ist, dass er mit dem Sensor-Körperteil (522, A522) in Kontakt kommt, um das Sensor-Körperteil (522, A522) zu stützen,
wobei die verbleibenden der mehreren elektrischen Kontakte (531, A531) so ausgebildet sind, dass sie von dem Sensor-Körperteil (522, A522) beabstandet sind,
wobei der mindestens eine der mehreren elektrischen Kontakte (531, A531), der das Sensor-Körperteil (522, A522) stützt, nicht mit einem Schaltungsmuster des PCB-Substrats (530, A530) verbunden ist, und
wobei das Sensor-Körperteil (522, A522) in einem von den verbleibenden der mehreren elektrischen Kontakte (531, A531) beabstandeten Zustand dazu ausgebildet ist, durch Betätigung durch einen Benutzer mit allen der mehreren elektrischen Kontakte (531, A531) in Kontakt zu kommen.

2. Am Körper anbringbare Einheit (20, A20) nach Anspruch 1, die ferner ein Druckbetätigungsmodul (570, A570) aufweist, das mit dem Gehäuse (510, A510) gekoppelt ist, um durch Betätigung durch den Benutzer betätigt zu werden, und dazu ausgebildet ist, das Sensor-Körperteil (522, A522) so zu drücken, dass das Sensor-Körperteil (522, A522) mit allen der mehreren elektrischen Kontakte (531, A531) in Kontakt kommt.

3. Am Körper anbringbare Einheit (20, A20) nach Anspruch 2, wobei das Sensor-Körperteil (522, A522) dazu ausgebildet ist, durch Drücken des Druckbetätigungsmoduls (570, A570) verformt zu werden, um mit allen der mehreren elektrischen Kontakte (531, A531) in Kontakt zu kommen.

4. Am Körper anbringbare Einheit (20, A20) nach Anspruch 3, wobei ein elektrischer Kontakt (531, A531) unter den elektrischen Kontakten (531, A531), der mit dem Sensor-Körperteil (522, A522) in Kontakt kommt, um das Sensor-Körperteil (522, A522) zu stützen, dazu ausgebildet ist, elastisch verformt zu werden, während ein Zustand des Herstellens eines Kontakts mit dem Sensor-Körperteil (522, A522) aufrechterhalten wird, wenn das Sensor-Körperteil (522, A522) durch das Druckbetätigungsmodul (570, A570) verformt wird.

5. Am Körper anbringbare Einheit (20, A20) nach Anspruch 1, wobei die elektrischen Kontakte (531, A531) so ausgebildet sind, dass sie von dem PCB-Substrat (530, A530) in einer elastisch verformbaren Form elastisch vorstehen.

6. Am Körper anbringbare Einheit (20, A20) nach Anspruch 5, wobei ein elektrischer Kontakt (531, A531), der mit dem Sensor-Körperteil (522, A522) in Kontakt kommt, um das Sensor-Körperteil (522, A522) zu stützen, unter den elektrischen Kontakten (531, A531), so ausgebildet ist, dass er eine Vorsprungshöhe von dem PCB-Substrat (530, A530) aufweist, die höher ist als die der verbleibenden der mehreren elektrischen Kontakte (531, A531).

## Revendications

1. Unité de fixation corporelle (20, A20), prévue pour être fixée et insérée dans un corps afin de mesurer des informations biométriques, ladite unité de fixation corporelle (20, A20) comprenant :
un boîtier (510, A510) formé de sorte qu'une surface inférieure est prévue pour être fixée sur la peau ;
un substrat de carte de circuit imprimé, PCB, (530, A530), disposé dans le boîtier (510, A510), avec une pluralité de contacts électriques (531, A531) formés en saillie d'une surface dudit substrat de PCB (530, A530) ; et
un élément de capteur (520, A520) comprenant une partie de corps de capteur (522, A522) disposée dans le boîtier (510, A510) et une partie de sonde de capteur (521, A521) formée et s'étendant depuis un côté de la partie de corps de capteur (522, A522) de manière à faire saillie vers l'extérieur depuis la surface inférieure du boîtier (510, A510), et prévue pour être insérée dans la peau lorsque le boîtier (510, A510) est fixé sur la peau,
où au moins un contact de la pluralité de contacts électriques (531, A531) est formé de manière à établir un contact avec la partie de corps de capteur (522, A522) afin de supporter la partie de corps de capteur (522, A522),
où les contacts restants de la pluralité de contacts électriques (531, A531) sont formés de manière à être espacés de la partie de corps de capteur (522, A522),
où ledit au moins un contact de la pluralité de contacts électriques (531, A531) supportant la partie de corps de capteur (522, A522) n'est pas connecté à un circuit à motif du substrat de PCB (530, A530), et
la partie de corps de capteur (522, A522), en état d'espacement des contacts restants de la pluralité de contacts électriques (531, A531), est prévue pour établir un contact avec tous les contacts de la pluralité de contacts électriques (531, A531) par manipulation d'un utilisateur.

2. Unité de fixation corporelle (20, A20) selon la revendication 1, comprenant en outre un module d'actionnement de pression (570, A570) raccordé au boîtier (510, A510) et devant être actionné par manipulation de l'utilisateur, et prévu pour comprimer la partie de corps de capteur (522, A522), de sorte que ladite partie de corps de capteur (522, A522) établit un contact avec tous les contacts de la pluralité de contacts électriques (531, A531).

3. Unité de fixation corporelle (20, A20) selon la revendication 2, où la partie de corps de capteur (522, A522) est prévue pour être déformée par pression du module d'actionnement de pression (570, A570) afin d'établir un contact avec tous les contacts de la pluralité de contacts électriques (531, A531).

4. Unité de fixation corporelle (20, A20) selon la revendication 3, où un contact électrique (531, A531) venant en contact avec la partie de corps de capteur (522, A522) pour supporter la partie de corps de capteur (522, A522) parmi les contacts électriques (531, A531) est prévu pour être déformé élastiquement en maintenant un état de contact avec la partie de corps de capteur (522, A522) lorsque la partie de corps de capteur (522, A522) est déformée par le module d'actionnement de pression (570, A570).

5. Unité de fixation corporelle (20, A20) selon la revendication 1, où les contacts électriques (531, A531) sont formés de manière à faire saillie élastiquement du substrat de PCB (530, A530) sous une forme déformable élastiquement.

6. Unité de fixation corporelle (20, A20) selon la revendication 5, où un contact électrique (531, A531) venant en contact avec la partie de corps de capteur (522, A522) pour supporter la partie de corps de capteur (522, A522) parmi les contacts électriques (531, A531) est formé de manière à présenter une hauteur de saillie du substrat de PCB (530, A530) supérieure aux contacts restants de la pluralité de contacts électriques (531, A531).
